# EUROPEAN PATENT SPECIFICATION

(11) **EP 0 880 972 B1**
(45) Date of publication and mention of the grant of the patent: **25.02.2004**
(21) Application number: 98107826.4
(22) Date of filing: 29.04.1998
(51) Int. Cl.: A61L 2/20, A61L 2/24

(54) **Apparatus and method of sterilization**
Vorrichtung und Verfahren zum Sterilisieren
Dispositif et procédé de stérilisation

(30) Priority: 29.05.1997 JP 15585397
(43) Date of publication of application: 02.12.1998
(73) Proprietor: SHIBUYA KOGYO CO., LTD, Kanazawa-shi, Ishikawa-ken (JP)
(72) Inventor: Nakahira, Kobun, c/o Shibuya Kogyo Co., Ltd., Kanazawa-shi, Ishikawa-ken (JP)
(74) Representative: Füchsle, Klaus, Dipl.-Ing.

(56) References cited:
- EP-A- 0 298 694
- EP-A- 0 758 611
- EP-A- 0 774 263
- GB-A- 2 127 692
- US-A- 5 229 071

## Description

The invention relates to an apparatus for and a method of sterilization which takes place by containing an object or article in a sterilizing chamber, into which a sterilizing gas is fed for purpose of sterilization. Such an apparatus and such a method are known from EP 0 298 694 A2.

A sterilization by way of a sterilizing gas has been used in the conventional practice for a container formed of a resin, for example, such as an injection syringe or eyedropper for which steam or dry hot sterilization cannot be used.

Such a gas sterilizer (see Japanese Patent Publications No.9,456/1985 and No.36,541/1984) generally comprises a sterilizing chamber which can be sealingly enclosed to contain an article therein, a sterilizing gas feeder connected to the chamber through a valve for feeding a sterilizing gas thereto, a vacuum pump for drawing gas from the chamber, and an air inlet connected to the chamber through a valve for introducing sterilized air which is passed through a sterilizing filter into the chamber. Normally, an article to be sterilized is received in the chamber, which is then drawn by the vacuum pump to achieve a vacuum condition at a given pressure within the chamber. The sterilizing gas feeder then feeds the gas into the chamber for permeation or immersion of the article for purpose of sterilization.

After the sterilization, the vacuum pump draws sterilizing gas from the chamber to be displaced therefrom, followed by the introduction of sterilized air into the chamber. By repeating the steps of drawing by the pump and introducing sterilized air, sterilizing gas is removed from the chamber, and the sterilized article is then removed from the chamber.

The sterilizers use ethylene oxide gas or the like as the sterilizing gas. The use of ethylene oxide gas for sterilization involves a problem that a sterilization cycle becomes longer. In particular, it takes a long time for a degassing step or displacement of sterilizing gas from the chamber upon completion of the sterilization process. In addition, ethylene oxide gas is strongly toxic, causing adverse influences upon men and global environment. The influences upon men and global environment are even more significant inasmuch as toxicity remains in decomposed products from the sterilization process.

To avoid the length of time required for the degassing step which follows the sterilization process when ethylene oxide gas is used as sterilizing gas, there has been proposed to connect a circulation path, which forms a closed circuit, to a sterilizing chamber in order to allow sterilizing gas to be displaced from the sterilizing chamber within a reduced length of time. The air which is fed into the chamber after the vacuum condition is established therein is passed through the circulation path, thus circulating it (Japanese Patent Publication No. 9,456/1985). However, the air circulation through the path to mix sterilizing gas and air to promote the fluidity is insufficient to accelerate the degassing step.

A similar method is known from EP 0 774 263 A1.

### OBJECT AND SUMMARY OF THE INVENTION

Accordingly, it is an object of the invention to provide an apparatus for and a method of sterilization which are capable of reducing the time required for the sterilization cycle by accelerating the degassing step which follows the sterilization while avoiding adverse influences upon human bodies and environment.

This object is solved by an apparatus having the features of claim 1, and by a method having the features of claim 4, respectively. The dependent claims define further advantageous embodiments.

When the method of sterilization is carried out using the apparatus for sterilization, hydrogen peroxide gas is fed to the sterilizing chamber which is maintained vacuum by operation of the vacuum pump, thereby sterilizing the article contained in the chamber. The step of drawing vacuum and the step of feeding hydrogen peroxide are alternately repeated to achieve a perfect sterilization. Subsequently, air is introduced into the chamber, and a gas mixture comprising the air and the hydrogen peroxide gas is circulated through the closed circuit circulation path, whereupon the hydrogen peroxide gas in the mixture is adsorped by the catalyst, thus reducing the concentration of hydrogen peroxide in the mixture gas.

### BRIEF DESCRIPTION OF THE DRAWING

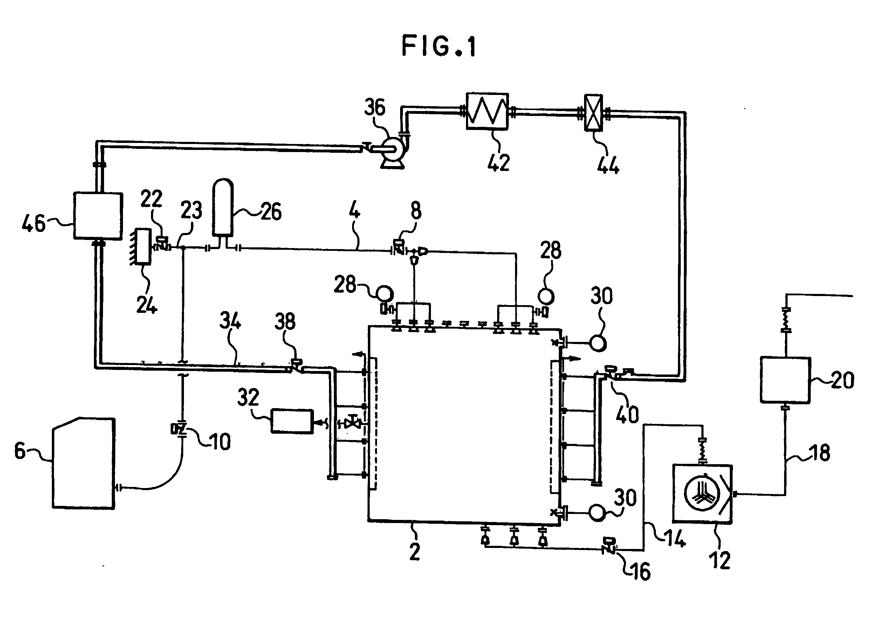

Fig. 1 is a circuit diagram schematically illustrating an apparatus for sterilization according to one embodiment of the invention.

### DETAILED DESCRIPTION OF PREFERRED EMBODIMENT

An embodiment of the invention will now be described with reference to the single drawing, which is a circuit diagram schematically illustrating the apparatus for sterilization according to one embodiment of the invention. A sterilizing chamber 2, which contains an article therein for sterilization, is connected through a gas pipe 4 to a hydrogen peroxide gas feeder 6. Valves 8 and 10 are disposed in the gas pipe 4 toward the chamber 2 and the feeder 6, respectively.

A vacuum pump 12, operative to draw gas from the chamber 2, is connected to the chamber 2 through a suction pipe 14 and a valve 16. Gas drawn by the pump 12 is passed through a downstream discharge pipe 18 having a catalyst 20 disposed therein to be exhausted externally or outdoors. An air inlet 24 is connected to a point in the gas pipe 4 located between the pair of valves 8, 10 through another valve 22 and an extension pipe 23. Air admitted through the air inlet 24 is introduced into the chamber 2 through a sterilizing filter 26 which is connected in the gas pipe 4. A pressure gauge 28 and a thermometer 30 are mounted on the chamber 2 to detect the pressure and the temperature within the chamber. A concentration meter 32 is also mounted to determine the concentration of hydrogen peroxide gas in the chamber 2.

A circulation path 34 is connected to the chamber 2 and forms a closed circuit together with the latter. A circulating fan 36 is connected in the circulation path 34, and valves 38 and 40 are also connected in the circulation path 34 at locations toward the outlet and the inlet, respectively, of the chamber 2. When the both valves 38 and 40 are open, the operation of the fan 36 allows gas in the chamber 2 to be circulated. To promote decomposition of hydrogen peroxide gas, a heater 42 which heats the circulating gas and a filter 44 are connected in the circulation path 34. A catalyst 46 is also disposed in the circulation path 34 to adsorp hydrogen peroxide gas contained in the air which circulates the path 34. In the present embodiment, platinum or palladium is used for the catalyst 46, whereby a simple passage of hydrogen peroxide therethrough enables a decomposition thereof in a facilitated manner.

A sterilization process which takes place with the apparatus for sterilization mentioned above will be described. Initially, an article to be sterilized is placed in the chamber 2, which is then sealingly enclosed by closing an access door. At this point, the interior of the chamber 2 assumes an atmospheric pressure.

The valve 16 connected in the suction pipe 14 connected to the vacuum pump 12 is then opened, and the pump 12 is operated, thus drawing air from the chamber 2 to achieve a vacuum condition. When a vacuum condition which corresponds to a given pressure is attained, the valve 16 associated with the pump 12 is closed while the valves 8 and 10 in the gas pipe 4 are opened to begin and continue supplying hydrogen peroxide gas from the feeder 6 into the chamber 2 until the entire article to be sterilized is immersed in the hydrogen peroxide gas. It is to be noted that a chosen amount of hydrogen peroxide gas be supplied since when supplied in excess, the hydrogen peroxide no longer assumes a gas phase. It is also to be noted that since the air pressure in the chamber 2 is greatly reduced as a result of operation of the vacuum pump 12, the hydrogen peroxide gas has a high level of partial pressure.

Subsequently, the valve 22 associated with the air inlet 24 is opened to allow sterilized air, which is obtained by passage through the sterilizing filter 26, to be introduced into the chamber 2, raising the internal pressure thereof slightly above its initial vacuum condition. This condition is maintained for a few minutes, allowing the hydrogen peroxide gas to permeate into detailed features of the article. Sterilization is then continued by repeating a sequence of the suction step by the pump 12, the step of feed sterilizing gas and the step of introducing sterilized air several times.

After the article is sterilized by the steps mentioned above, the degassing step to remove hydrogen peroxide gas from the chamber 2 takes place. Again the vacuum pump 12 is operated to reestablish a vacuum condition corresponding to a given pressure within the sterilizing chamber 2 by drawing gas therefrom. After the vacuum condition is attained, the valve 16 associated with the pump 12 is closed while the valve 22 associated with the air inlet 24 and the valve 8 disposed in the gas pipe 4 toward the chamber 2 are opened, thus introducing sterilized air into the chamber 2 and raising the pressure within the chamber to a level slightly below the atmospheric pressure.

The valves 22 and 8 are then closed while the valves 38 and 40 connected in the circulation path 34 toward the outlet and the inlet of the chamber 2 are opened, and the circulating fan 36 is operated. The gas mixture of the air and the hydrogen peroxide gas circulates through the path 34. During the circulation, the catalyst 46 such as platinum adsorps the hydrogen peroxide and the heating of the circulating gas by the heater 42 promotes the decomposition of the hydrogen peroxide gas, thus reducing the concentration of the hydrogen peroxide in the chamber 2. When the gas circulation through the path 34 is continued for a given time interval, the concentration of the hydrogen peroxide will be significantly reduced. In this manner, by causing a circulation of an increased volume of air through the catalyst 46 subsequent to the sterilization, the decomposition and the removal of the hydrogen peroxide gas can be achieved in a reduced length of time. When the hydrogen peroxide gas is removed from the chamber 2, an access door of the chamber is opened to remove the article which is now sterilized. In this embodiment, the sterilization is achieved by using hydrogen peroxide gas which is not toxic. Accordingly, there is no adverse influence upon human bodies and environment, and decomposition products from the sterilization are oxygen and water, which are harmless.

Alternatively, subsequent to the removal of the hydrogen peroxide gas through the air circulation through the circulation path 34, the valve 16 in the pipe 14 leading to the pump 12 as well as the valve 22 in the extension pipe 23 from the air inlet 224 and the valve 8 in the gas pipe may be opened while simultaneously operating the pump 12, thus continuously displacing gas through the chamber 2 while drawing the air through the air inlet 24.

In the apparatus of the embodiment, when placing or removing an article into or from the chamber 2, the access door of the chamber may be maintained fully open while the pump 12 is operated to displace gas, thus preventing any residue of hydrogen peroxide gas in the chamber 2 from flowing out of the chamber. It is to be understood that articles which can be sterilized by the apparatus of the embodiment are not limited to resin containers such as injection syringe or eyedropper, but that the invention is applicable to a variety of articles. The invention is particularly preferred for use with articles which are not heat resistant, and a perfect sterilization is assured even for an article which has a complicated configuration.

## Claims

1. An apparatus for sterilization comprising a sterilizing chamber (2) capable of containing an article therein, a hydrogen peroxide feeder (6) connected to the chamber through a gas pipe (4), a vacuum pump (12) connected to the chamber through a suction pipe (14) for drawing gas therefrom, and an air inlet (24) connected to the chamber (2) through a filter (26), **characterized by** a closed circuit circulation path (34) connected to the chamber (21) through an outlet valve (38) and an inlet valve (40), a circulating fan (36) provided in the closed circuit circulation path, and a catalyst (46) disposed in the closed circuit circulation path.

2. An apparatus according to Claim 1 in which a heater (42) is provided in the circulation path (34).

3. An apparatus according to Claim 1 or 2 in which a filter (44) is provided in the circulation path (34).

4. A method of sterilization comprising the steps of using a vacuum pump to draw gas from a sterilizing chamber which is sealingly enclosed to contain an article therein, feeding hydrogen peroxide gas into the sterilizing chamber for sterilization, repeating the steps of drawing gas and feeding hydrogen peroxide gas a plurality of times, subsequently introducing sterilized air into the chamber, **characterized by** circulating gas in the chamber by means of a circulating fan and through a closed circuit circulation path which is connected to the chamber through an inlet valve and an outlet valve, and passing the gas through a catalyst which is disposed in the circulation path to remove the hydrogen peroxide gas.

5. A method according to Claim 4 in which the step of feeding hydrogen peroxide gas into the sterilizing chamber for sterilization comprises subsequent to feeding the hydrogen peroxide gas, introducing sterilized air into the sterilizing chamber, this condition being maintained for a given time interval.

6. A method according to Claim 4, further comprising subsequent to the step of removing the hydrogen peroxide gas, applying a suction to the sterilizing chamber from the vacuum pump while introducing air from an air inlet into the sterilizing chamber.

7. A method according to Claim 4, further comprising the step of operating the vacuum pump to displace gas when an article is placed into or removed from the sterilizing chamber.

## Patentansprüche

1. Einrichtung zur Sterilisierung, die eine Sterilisierungskammer (2) aufwiest, in welcher ein Gegenstand aufgenommen werden kann, eine Zufuhrvorrichtung (6) für Wasserstoffperoxyd, die an die Kammer über ein Gasrohr (4) angeschlossen ist, eine Vakuumpumpe (12), die an die Kammer durch ein Saugrohr (14) angeschlossen ist, um aus dieser Gas abzuziehen, und einen Lufteinlass (24), der an die Kammer (2) über eine Filterkammer (26) angeschlossen ist, **gekennzeichnet durch** einen geschlossenen Umwälzungsweg (34), der an die Kammer (21) **durch** ein Auslassventil (38) und ein Einlassventil (40) angeschlossen ist, ein in dem geschlossenen Umwälzungsweg vorgesehenes Umwälzgebläse (36), und einen in dem geschlossenen Umwälzungsweg angeordneten Katalysator (46).

2. Einrichtung nach Anspruch 1, bei welcher eine Heizvorrichtung (42) in dem Umwälzungsweg (34) vorgesehen ist.

3. Einrichtung nach Anspruch 1 oder 2, bei welcher ein Filter (44) in dem Umwälzungsweg (34) vorgesehen ist.

4. Verfahren zur Sterilisierung, mit den Schritten, eine Vakuumpumpe zum Abziehen von Gas von einer Sterilisierungskammer zu verwenden, die abgedichtet umschlossen ist, damit in ihr ein Gegenstand aufgenommen werden kann, Zuführen von Wasserstoffperoxydgas in die Sterilisierungskammer zur Sterilisierung, Wiederholen der Schritte des Abziehens von Gas und des Zuführens von Wasserstoffperoxydgas mehrfach, nachfolgendes Einlassen von sterilisierter Luft in die Kammer, **gekennzeichnet durch** Umwälzen von Gas in der Kammer mithilfe eines Umwälzgebläses, und **durch** einen geschlossenen Umwälzungsweg, der an die Kammer **durch** ein Einlassventil und ein Auslassventil angeschlossen ist, und Durchleiten des Gases **durch** einen Katalysator, der in dem Umwälzungsweg angeordnet ist, um das Wasserstoffperoxydgas zu entfernen.

5. Verfahren nach Anspruch 4, bei welchem der Schritt der Zuführung von Wasserstoffperoxydgas in die Sterilisierungskammer zur Sterilisierung umfasst, nach dem Zuführen des Wasserstoffperoxydgases sterilisierte Luft in die Sterilisierungskammer einzulassen, wobei dieser Zustand über ein vorgegebenes Zeitintervall beibehalten wird.

6. Verfahren nach Anspruch 4, welches folgend auf den Schritt des Entfernens des Wasserstoffperoxydgases umfasst, eine Saugwirkung auf die Sterilisierungskammer von der Vakuumpumpe aufzubringen, während Luft von einem Einlass in die Sterilisierungskammer eingelassen wird.

7. Verfahren nach Anspruch 4, mit dem weiteren Schritt, die Vakuumpumpe dazu zu betreiben, dass Gas verdrängt wird, wenn ein Gegenstand in die Sterilisierungskammer eingebracht oder aus dieser entnommen wird.

## Revendications

1. Dispositif de stérilisation comprenant une chambre de stérilisation (2) pouvant contenir un article en son intérieur, un alimentateur en peroxyde d'hydrogène (6) relié à la chambre par l'intermédiaire d'un tuyau à gaz (4), une pompe à vide (12) reliée à la chambre par l'intermédiaire d'un tuyau d'aspiration (14) pour aspirer le gaz de celle-ci, et une entrée d'air (24) reliée à la chambre (2) par l'intermédiaire d'un filtre (26), **caractérisé par**, un chemin de circulation à circuit fermé (34) relié à la chambre (21) par l'intermédiaire d'une vanne de sortie (35) et d'une vanne d'entrée (40), un ventilateur brasseur d'air (36) fourni dans le chemin de circulation à circuit fermé, et un catalyseur (46) disposé dans le chemin de circulation à circuit fermé.

2. Dispositif selon la revendication 1, dans lequel un appareil de chauffage (42) est fourni dans le chemin de circulation (34).

3. Dispositif selon la revendication 1 ou 2, dans lequel un filtre (44) est fourni dans le chemin de circulation (34).

4. Procédé de stérilisation comprenant les étapes consistant à utiliser une pompe à vide pour aspirer du gaz depuis une chambre de stérilisation qui est incorporée de manière étanche afin de contenir un article en son intérieur, alimenter du gaz peroxyde d'hydrogène dans la chambre de stérilisation pour stérilisation, répéter les étapes consistant à aspirer du gaz et à alimenter du gaz peroxyde d'oxygène une pluralité de fois, introduire par la suite de l'air stérilisé dans la chambre, **caractérisé par** le fait de faire circuler du gaz dans la chambre au moyen d'un ventilateur brasseur d'air et par l'intermédiaire d'un chemin de circulation à circuit fermé qui est relié à la chambre par l'intermédiaire d'une valve d'entrée et d'une valve de sortie, et à faire passer le gaz par l'intermédiaire d'un catalyseur qui est disposé dans le chemin de circulation pour retirer le gaz de peroxyde d'hydrogène.

5. Procédé selon la revendication 4, dans lequel l'étape consistant à alimenter du gaz de peroxyde d'hydrogène dans la chambre de stérilisation pour la stérilisation comprend à la suite de l'étape d'alimentation en gaz de peroxyde d'hydrogène le fait d'introduire de l'air stérilisé dans la chambre de stérilisation, cette condition étant maintenue pour un intervalle de temps donné.

6. Procédé selon la revendication 4, comprenant en outre après l'étape consistant à retirer le gaz de peroxyde d'hydrogène, l'étape consistant à appliquer une aspiration à la chambre de stérilisation depuis une pompe à vide tout en introduisant de l'air depuis une entrée d'air dans la chambre de stérilisation.

7. Procédé selon la revendication 4, comprenant en outre l'étape consistant à faire fonctionner la pompe à vide pour déplacer le gaz lorsqu'un article est placé dans. ou retiré de la chambre de stérilisation.
